# EUROPEAN PATENT APPLICATION

(11) **EP 0 906 952 A2**
(43) Date of publication of application: **07.04.1999**
(21) Application number: 98850145.8
(22) Date of filing: 18.09.1998
(51) Int. Cl.: C12N 1/20, A23K 3/00

(54) **Bacterial strain for use in ensiling of straw fodder**

(30) Priority: 26.09.1997 SE 9703492
(71) Applicant: Medipharm AB, 260 23 Kageröd (SE)
(72) Inventor: Lindgren, Sven, S-753 13 Uppsala (SE); Roos, Stefan, S-757 54 Uppsala (SE)
(74) Representative: Avellan-Hultman, Olle

(57) **Abstract**

The invention relates to a bacterial strain of the type *Lactococcus* which restrains the origin of yeast and clostrides and mould, and gram positive bacteria and certain gram negative bacteria and which exhibits good survivance, and which bacterial strain is the strain *Lactococcus lactis* subsp. *lactis* which is deposited with the National Collections of Industrial and Marine Bacteria Ltd, Aberdeen, Scotland, under the deposition number NCIMB 30117. The invention also relates to use of the bacterial strain *Lactococcus lactis* subsp. *lactis* in ensiling of green fodder/straw fodder without admitting air thereinto, or in combination with other preserving bacterial strains of known type, and eventually some enzyme.

## Description

The present invention relates to a bacterial strain which has proved to give good effects in connection to preserving of straw fodder.

Since long different types of straw fodder generally have been preserved in that the straw fodder, after having been harvested, has been allowed to dry in the air and has thereafter been stored in dry condition. In modern agriculture methods a technic has been developed for preserving straw fodder in damp condition. Said technic is built on the process of storing the fresh fodder, which has just been harvested or has been slightly pre-dried, is stored well packed in an air tight packing means. Under such conditions lactobacillus, which are naturally present in the straw fodder, are successively developed, and said lactobacillus produces lactic acid, which in turn provides an acid preserving of the fodder. The development of lacobacillus, however, follows relatively slowly, and this leads to conditions for growth of non desired micro organisms, for instance clostrides.

For improving and speeding up the preserving effect it is known to admix various types of additives into the fresh straw fodder, for instance formic acid, whereby the pH value of the straw fodder is optimized to about pH 4. Alternatively, or as a supplement to the admixing of formic acid there has, in some cases, been added molasses, lactobacillus or certain enzymes which have increased the availability of nutrients in the straw fodder. There also are chemical substances on the market which give a direct preserving effect.

A disadvantage in sole acidic preserving of straw fodder is that there may also develop other bacteria than the desired lactic acid bacteria, for instance spore forming organisms, generally referred to as clostrides. A high content of clostrides in the ensilage generally leads to the effect that the milk from the cow also gets a high content of clostrides. Since said clostrides are not killed at normal pasteurization processes dairy products made from such milk are negatively affected. For instance there may appear false fermentation in cheese products.

An object with the invention therefore has been to eliminate the disadvantages which appear in conventional processes of acidic preserving of green fodder, and especially to find a substance which is useful as a preserving additive in ensilage and which restrains the growth of clostrides and which does not promote a fermentation in the ensilage. It would be especially advantageous that such an preserving additive, in itself or by the influence of a substance produced by itself, impedes the origin and growth of clostrides and yeast, other gram-positive and certain gram-negative bacteria. The additive also should exhibit good survivance in the ensilage.

By extensive tests it has been possible to find a method for isolating a certain type of lactobacillus which exhibits the desired properties. In connection to tests with ensiling of lucerne certain starch hydrolysing lactobacillus were added. Said tests were successful and it was noted that the origin and growth of gram-positive bacteria were strongly restrained, for instance *Listeria monocytogenes, Staphylococcus aureus, Clostridium tyroburyticum, Bacillus cereus* and various lactic acid bacteria. Also the growth of certain gram-negative bacteria were restrained, even if relatively weakly, for instance *Pseudomonas aeruginosa.*

It could be established that reason for the restraining of the bacterial growth did not depend on the presence of an acid or of a hydrogen peroxide.

In the microbiological analysis of a check type ensilage at said tests, without addition of any starter culture, there were observed three different colonies in the growth medium in which the bacteria have been cultivated, which colonies strongly restrained the surrounding colonies. The bacteria were very resistant to both heat and protease treatment with protein splitting enzyme. It also could be established that the bacteria were resistant to the normally bacteria killing poly-peptide nisin. This could indicate that at least some of the isolated colonies produce nisin by themselves.

Two isolates which were supposed to produce nisin were detected by means of Polymerase Chain Reaction (PCT), the genes were sequenced and it proved that it was the gene variant nisin Z. The bacterial strains were identified by the microbiological laboratory BCCM/LMG at Gent, Belgium as being *Lactococcus lactis,* subsp. *lactis.* The identification was made using the protein design from the bacteria. The amount of G- and C-nucleotides in the genome from the isolated bacterial strain were analyzed. The two isolagenes had about 38% GC. It proved that the growth of bacteria was restrained by vancomycin, and this seems to indicate that the strains are not a lactobacillus but a lactococcus.

It also proved that the bacteria could be used for instance for impeding the origin of bacillus spores which can otherwise make milk become thickened.

The bacteria has been deposited with The National Collections of Industrial and Marine Bacteria Ltd, Aberdeen, Scotland, under the deposition number NCIMB 30117.

The bacteria has been prepared industrially in that a bud of a pure culture was admixed to a broth which has been sterilized in advance. The bacteria were thereafter cultured for 10-20 hours at a temperature which is an optimum temperature for the strain in question. The lactic acid which was formed during the culturing process was neutralized by an addition of NaOH for the purpose of maintaining the culture broth at an optimum pH value. When the supply of bacterial cells had reached a certain concentration the growth stops and the cultivation is finished. Thereafter a concentration follows by means of a separator or a filter, whereby the bacterial cells which have been obtained are separated from the cultivation broth.

The bacterial mass obtained can thereafter be freeze dried and can be supplied as a powder which can be solved in water and can be used for instance by being sprayed onto green fodder when said fodder is introduced in packages or in an ensilage tower. The bacteria preferably can be added in an amount of 4-8 litres per ton ensilage. When storing the ensilage in a tower said ensilage ought to be finely chopped and it should have a dry matter content of 20-45%.

Thus, the invention relates to a bacterial strain of the type *Lactococcus* for use as a starter culture in ensiling of green fodder, and which culture impedes the origin of gram positive bacteria, yeast and clostrides, and also certain gram negative bacteria in the ensilage and which exhibits a good survivance in the ensilage.

The invention is characterized in that the bacterial strain is *Lactococcus lactis* subsp. *lactis.* A specific feature is that the bacterial strain itself produces nisin.

The invention also relates to the use of the bacterial strain *Lactococcus lactis* subsp. *lactis* as a starter culture in ensiling of green fodder without admitting air thereinto, whereby a solution of the bacterial strain is admixed into the green fodder in an amount of 4-8 litres per ton green fodder having a dry matter content of 20-45%.

Preferably the bacterial strain *Lactococcus lactis* subsp. *lactis* according to the invention is used as an ingredient in a bacterial mixture comprising *Enterococcus faecium, Pediococcus acidilacti, Lactobacillus plantarum* and an amount of the enzyme cellulase. In the said bacterial mixture, which is marketed under the trade name "Lactisil Plus" there are four ingredients in substantially like parts and in a total amount of between 10⁵ and 10⁶ CFU per gram ensilage. According to the invention the bacteria *Lactococcus lactis* subsp. *lactis* is added to the previously used bacterial mixture in an amount of between 10⁴ and 10⁶ per gram ensilage, that is so that the total amount of preservative added to the mixture is between 110.000 and 2.000.000 CFU per gram ensilage.

### Tests

For judging the effect of the bacteria *Lactococcus lactis* subsp. *lactis* according to the invention, at ensiling of mixed green fodder there were made several tests, whereby the freeze dried bacteria according to the invention was solved in water and was admixed into the green fodder by means of spray nozzles in an amount corresponding to 4-8 litres per ton green fodder. The following tables 1, 2 and 3 show the mean values of the several tests. The tests were judged from three various aspects, namely a) in relation to untreated green fodder, b) in relation to a conventional additive named "Lactisil Plus" which additive, according to what is said above, is composed of lactic acid producing strains and an enzyme, and c) the same additive comprising a like amount of the bacteria *Lactococcus lactis* subsp. *lactis* according to the invention. In the following table 1 there are shown the storage losses in %, in table 2 there are shown the effect of microbiological growth of lactic bacteria, clostrides and yeast in the ensilage, and in table 3 there is shown the effect of the additive at an addition of a destructive flora.

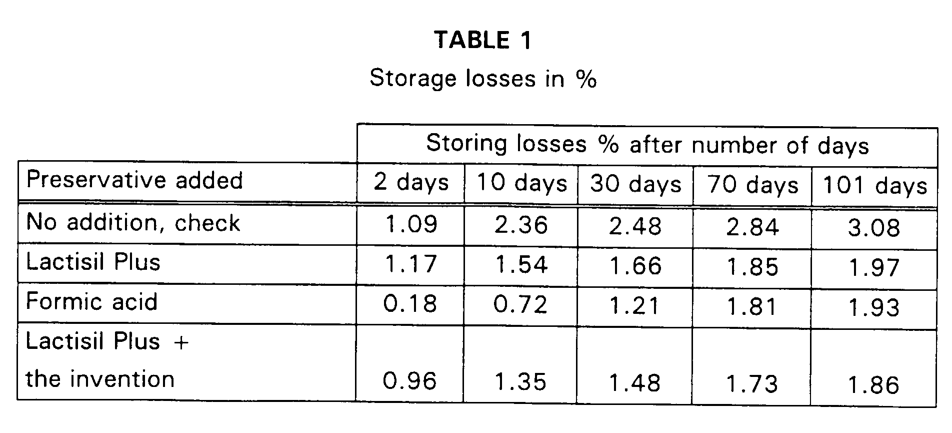

It is obvious that the addition of a preservative radically reduced the storing losses in ensilaged fodder. It is also seen from the table that the effect of the addition of *Lactobacillus lactis* subsp. *lactis* according to the invention is best observed at long time storing of the ensilage. For storing times of between 2 and 30 days formic acid is seemed to give a better effect than the addition according to the invention, but the situation is thereafter inversed. In addition hereto it should be observed that formic acid, as mentioned above, can lead to the origin of non wanted bacteria and clostrides. Further, formic acid is chemically aggressive and can be difficult to handle by the farmer.

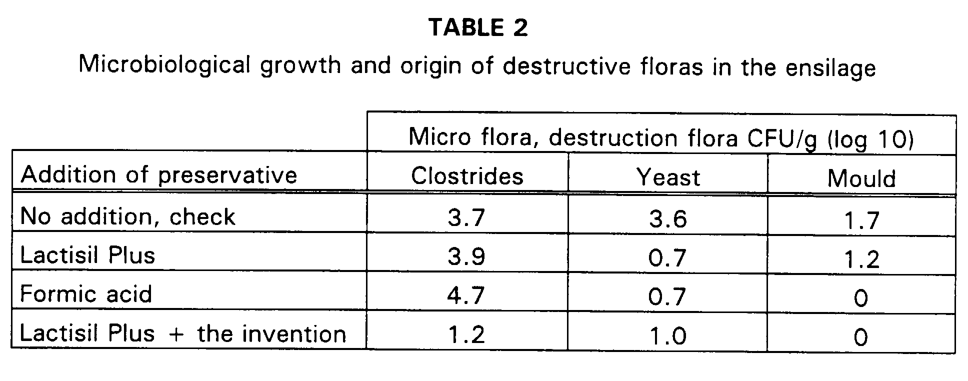

The checking of the content of clostrides, yeast and mould was made after the ensilage had been stored for 60 days. It is obvious from the above that an addition of the bacterial strain *Lactococcus lactis* subsp. *lactis* very markedly reduces the content of clostrides in the ensilage; the content of yeast is substantially less than in the check ensilage, but of the same magnitude independently which addition of preservative was used; no mould at all could be found, neither when using formic acid or adding the bacteria according to the invention. The content of clostrides at preserving the ensilage using the invention as an additive, however, is only about 0.03% of the corresponding content using formic acid as a preservative.

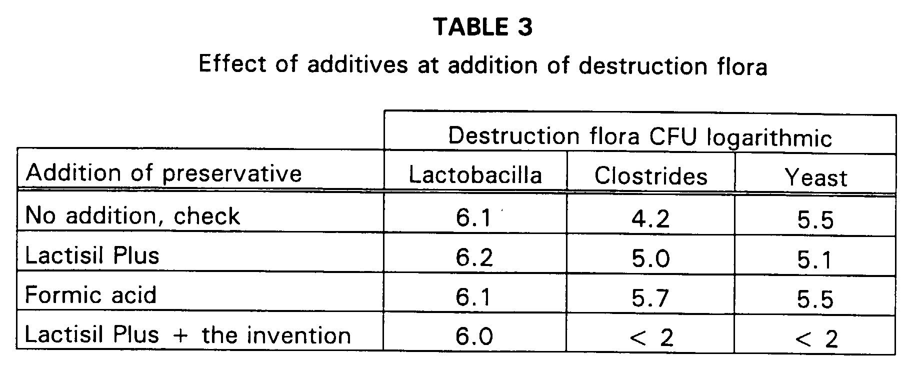

It is evident that the addition of bacteria according to the invention gives rise to a very little content of clostrides and of yeast in the ensilage, as compared with what is the case both for the check ensilage and at an addition of the known preservative Lactisil Plus and an addition of formic acid.

The shown good effect of the bacterial strain *Lactococcus lactis* subsp. *lactis* can, to a great extent, be supposed to appear in that the bacterial strain itself forms the polypeptide nisin and eventually some other substance which has a bacteria restraining effect, and which thereby restrains the origin of yeast, mould and clostrides already in small doses in the ensilage, like 100.000 CFU per gram ensilage.

## Claims

1. Bacterial strain of the type *Lactococcus* which restrains the origin of yeast and clostrides and mould, and which exhibits good survivance, characterized in that the bacterial strain is the strain *Lactococcus lactis* subsp. *lactis* which is deposited with the National Collections of Industrial and Marine Bacteria Ltd, Aberdeen, Scotland, under the deposition number NCIMB 30117.

2. Bacterial strain according to claim 1, **characterized** in that said strain is capable of forming lactic acid.

3. Bacterial strain according to claim 1 or 2, **characterized** in that said strain is capable of producing, itself, one of more substances which are capable of restraining the origin and growth of clostrides and yeast.

4. Bacterial strain according to any of claims 1, 2 or 3, **characterized** in that said strain is capable of producing, itself, nisin having a bacteria restraining effect.

5. Use of the bacterial strain according to any of claims 1 - 4 in ensiling green fodder/straw fodder, **characterized** in that the bacterial strain *Lactococcus lactis* subsp. *lactis* is admixed into the green fodder which is thereafter sealed under airtight conditions, whereby the admixed bacterial strain restrains the origin and growth of clostrides and yeast in the ensilage.

6. Use according to claim 5, **characterized** in that the bacterial strain *Lactococcus lactis* subsp. *lactis* is added to the fresh green fodder in an amount of between 10⁴ and 10⁶ CFU per gram green fodder.

7. Use according to claim 5 or 6, **characterized** in that the bacterial strain *Lactococcus lactis* subsp. *lactis* is admixed into the green fodder combined with other preservatives and with an amount of between 10% and 90% *Lactococcus lactis* subsp. *lactis* as calculated on the total amount of added preservatives.

8. Use according to claim 7, **characterized** in that the bacterial strain *Lactococcus lactis* subsp. *lactis* is mixed in amount of 10-90% with a bacterial mixture comprising *Enterococcus faecium, Pediococcus acidilacti, Lactobacillus plantarium* and an amount of the enzyme cellulase.
